# FASCICULE DE BREVET EUROPEEN

(11) **EP 2 996 642 B1**
(45) Date de publication et mention de la délivrance du brevet: **15.03.2017**
(21) Numéro de dépôt: 14727869.1
(22) Date de dépôt: 29.04.2014
(51) Int. Cl.: A61F 5/01, A61F 13/06

(54) **ORTHESE PROPRIOCEPTIVE ASSURANT LE MAINTIEN D'UNE ARTICULATION**
PROPRIOZEPTIVE ORTHESE ZUR UNTERSTÜTZUNG EINES GELENKS
PROPRIOCEPTIVE ORTHOSIS FOR SUPPORTING A JOINT

(30) Priorité: 16.05.2013 FR 1354401
(43) Date de publication de la demande: 23.03.2016
(73) Titulaire: Millet Innovation, 26270 Loriol sur Drome (FR)
(72) Inventeur: GRANGE, Odile, F-26400 Allex (FR); MILLET, Damien, F-26000 Valence (FR)
(74) Mandataire: de Roquemaurel, Bruno
(86) Numéro de dépôt international: PCT/FR2014/051019
(87) Numéro de publication internationale: WO 2014/184459

(56) Documents cités:
- EP-A1- 0 115 029
- DE-U1-202010 015 972
- FR-A1- 2 607 384
- FR-A1- 2 772 596
- FR-A1- 2 957 246
- US-A- 4 287 885
- US-A1- 2012 078 156

## Description

La présente invention concerne une orthèse proprioceptive destinée à assurer le maintien d'une articulation du corps humain. La présente invention s'applique notamment, mais non exclusivement au maintien d'une articulation telle que le genou ou le coude, ne nécessitant pas une immobilisation de l'articulation. Une telle orthèse peut être utilisée pour prévenir des douleurs chroniques, ou la suite d'une entorse légère, ou encore lors d'une reprise d'activité à la suite d'un traumatisme.

Les orthèses proprioceptives du genou se distinguent des genouillères traditionnelles par des aménagements tels que des "pelotes rotuliennes" destinées à assurer un certain maintien de la rotule, ou des pelotes de massage pour masser certains muscles. De telles orthèses sont décrites par exemple dans les demandes de brevet US 2006/0041214, US 2010/0036303 et US 2011/0160631, et le modèle DE 200 05 663 U1. Les orthèses décrites dans ces documents comprennent un élément annulaire en mousse, en silicone, en caoutchouc silicone ou en TPU (polyuréthane thermoplastique), destiné à entourer la rotule pour en assurer le maintien.

Ces orthèses présentent plusieurs défauts. Elles sont relativement épaisses et lourdes notamment parce qu'elles sont réalisées à l'aide de machines à tricoter qui permettent d'obtenir uniquement des tissus tricotés qui ne peuvent pas être aussi fins que les tissus tissés, c'est-à-dire comportant un fil de chaine et un fil de trame. En raison de la masse du tissu tricoté, relativement élevée, il peut être nécessaire de prévoir des armatures latérales pour éviter qu'elles s'effondrent sur elles-mêmes. En raison de leur épaisseur relativement importante et de la présence d'armatures, elles sont inconfortables sous un pantalon, voire inadaptées au port d'un pantalon étroit. Lors de flexions répétées du genou, par exemple en situation de course, elles ont tendance à glisser le long de la cuisse et de la jambe, notamment en raison de leur masse. Dans le modèle DE 20 2010 015 972 U1, sur lequel est basé le préambule de la revendication 1 annexée, il est prévu des surfaces antidérapantes dans la zone de l'articulation et aux extrémités de l'orthèse pour éviter ces glissements.

En situation de genou fléchi, les plis qui se forment inévitablement dans le pli poplité, à l'arrière du genou, se superposent et peuvent former une épaisseur de plusieurs millimètres, ce qui peut entraîner une gêne de l'utilisateur, voire des douleurs. Elles nécessitent souvent l'usage de bandes de serrage avec boucles et crochets pour assurer un maintien suffisant sur la cuisse et sous le genou.

Par ailleurs, il est également connu d'utiliser des plaquettes à base de gel polymère tel que du gel de silicone ou à base d'hydrogel, pour assurer une fonction de protection de la peau ou de répartition de charge. Par exemple pour assurer une fonction de répartition de charge, il est connu d'utiliser une plaquette en un gel de silicone de type PDMS (polydiméthylsiloxane) relativement dure. Le brevet FR 2 712 487 décrit un gel de silicone ayant des propriétés se rapprochant de celles du capiton plantaire pour la prévention de pathologies d'hyper appuis apparaissant essentiellement sur ou sous les pieds.

Il est souhaitable de réaliser une orthèse de faible épaisseur capable notamment de soulager les articulations, tout en assurant un maintien de l'orthèse en position. Il est également souhaitable de pouvoir ajuster les forces exercées par l'orthèse sur la peau et sur des tissus sous-jacents, en particulier à proximité d'une articulation.

Ces buts sont atteints par une orthèse selon la présente invention, définie dans la revendication indépendante 1. Un procédé de fabrication d'une telle orthèse est défini dans la revendication indépendante 13. Différents modes de réalisation sont définis dans les revendications dépendantes.

Ainsi, l'orthèse selon la présente invention comprend un manchon en un tissu tissé élastique, conformé pour exercer des forces de contention sur un membre de part et d'autre et sur une articulation, et une plaquette comprenant une couche en un gel polymère viscoélastique fixée sur une face interne du manchon pour venir en contact direct avec la peau sur l'articulation. Selon l'invention, la plaquette comprend une couche en un tissu élastique sur laquelle est collée la couche en gel polymère, une partie annulaire conformée pour entourer le sommet d'une zone proéminente de l'articulation, et une languette s'étendant depuis un bord extérieur de la partie annulaire, dans une direction axiale du manchon, la plaquette présentant une adhérence avec la peau telle que, sous l'effet des forces de contention exercées par le manchon, lorsque le manchon est étiré longitudinalement, elle reste étirée et applique localement à la partie de membre sous-jacente des forces de maintien en direction du centre de la zone proéminente de l'articulation, et des forces de rappel dans l'axe du membre.

Selon un mode de réalisation, la plaquette est fixée au manchon seulement par un secteur proximal de la partie annulaire et par une partie incluant un secteur distal de la partie annulaire et la languette.

Selon un mode de réalisation, la plaquette est fixée au manchon de manière à laisser libre des bords latéraux externes de la partie annulaire de la plaquette.

Selon un mode de réalisation, la plaquette est fixée au manchon le long d'un bord intérieur de la partie annulaire.

Selon un mode de réalisation, l'orthèse comprend deux coussinets latéraux comprenant une couche gel polymère viscoélastique et présentant la forme de secteurs latéraux de la plaquette fixées par des lignes d'assemblage s'étendant radialement par rapport à la plaquette, sous des secteurs latéraux de la plaquette, des bords latéraux des coussinets étant laissés libres.

Selon un mode de réalisation, la partie annulaire de la plaquette présente une ouverture coïncidant avec une première ouverture formée dans le manchon, l'ouverture étant refermée par une pièce en un tissu tissé élastique d'épaisseur inférieure à 0,3 mm fixée sur le manchon et/ou sur la plaquette.

Selon un mode de réalisation, le manchon comprend une seconde ouverture dans une zone diamétralement opposée à la plaquette et refermée par une pièce en un tissu tissé élastique d'épaisseur inférieure à 0,3 mm.

Selon un mode de réalisation, la pièce refermant la seconde ouverture formée dans le manchon, est fixée au manchon de manière à rester tendue quelle que soit la flexion du genou.

Selon un mode de réalisation, le manchon comporte le long de bords proximal et distal, sur sa face destinée à venir en contact avec la peau, des éléments adhérents pour contribuer au maintien du manchon sur le membre.

Selon un mode de réalisation, les éléments adhérents sont formés sur des bandes, fixées aux bords proximal et distal du manchon, les bandes étant réalisées en un tissu élastique pouvant être le même que celui du manchon.

Selon un mode de réalisation, la bande d'éléments adhérents fixée au bord proximal du manchon comprend deux bandes fixées l'une à l'autre et de longueurs différentes pour s'adapter à la forme du membre.

Selon un mode de réalisation, le manchon présente une épaisseur inférieure à 0,5 mm, et la plaquette une épaisseur inférieure à 1 mm.

Un procédé de fabrication d'une orthèse selon l'invention comprend des étapes consistant à : former un manchon en un tissu tissé élastique, apte à exercer des forces de contention sur un membre de part et d'autre et sur une articulation, former une plaquette comprenant une couche en un gel polymère viscoélastique, une couche en un tissu élastique collée sur la couche en gel polymère, une partie annulaire conformée pour entourer le sommet de l'articulation en position fléchie, et une languette s'étendant depuis un bord extérieur de la partie annulaire, et fixer la plaquette sur une face interne du manchon pour venir en contact direct avec la peau sur l'articulation, la languette étant orientée dans une direction distale du manchon, la plaquette présentant une adhérence avec la peau telle que, sous l'effet des forces de contention exercées par le manchon, lorsque le manchon est étiré longitudinalement, elle reste étirée et applique localement à la peau des forces de traction parallèles à la surface de la peau, en direction du centre de la partie annulaire.

Selon un mode de réalisation, la plaquette est fixée au manchon par une couture de manière à ce que des parties latérales des bords externes de la partie annulaire soient laissées libres.

Selon un mode de réalisation, le procédé de fabrication comprend une étape de fixation par des lignes d'assemblage s'étendant radialement par rapport à la plaquette, de coussinets comprenant une couche en gel polymère viscoélastique présentant la forme de secteurs latéraux de la plaquette, sous des parties latérales de la partie annulaire de la plaquette, des bords latéraux des coussinets étant laissés libres.

Selon un mode de réalisation, le procédé de fabrication comprend une étape de fixation d'une pièce de tissu tissé élastique pour refermer l'ouverture de la partie annulaire de la plaquette, la pièce de tissu présentant une épaisseur inférieure à 0,3 mm.

Selon un mode de réalisation, le procédé de fabrication comprend des étapes de formation d'une ouverture dans le manchon, en regard de la plaquette, et de fixation d'une pièce de tissu tissé élastique pour refermer l'ouverture formée dans le manchon, la pièce de tissu présentant une épaisseur inférieure à 0,3 mm.

Selon un mode de réalisation, la pièce refermant la seconde ouverture formée dans le manchon, est fixée au manchon de manière à rester tendue quelle que soit la flexion du genou.

Selon un mode de réalisation, le procédé de fabrication comprend une étape de moulage de la plaquette, le moule formant des picots sur la face de la languette destinée à venir en contact avec la peau, pour augmenter l'adhérence de la languette sur la peau.

Selon un mode de réalisation, le procédé de fabrication comprend des étapes de fixation le long des bords proximal et distal du manchon de bandes en tissu élastique, comportant des éléments adhérents pour contribuer au maintien du manchon sur le membre.

Des exemples de réalisation de l'invention seront décrits dans ce qui suit, à titre non limitatif en relation avec les figures jointes parmi lesquelles :
la figure 1 est une vue de face d'une orthèse de genou, selon un mode de réalisation,
la figure 2 est une vue de face de l'orthèse de la figure 1, placée sur un membre inférieur droit,
la figure 3 est une vue arrière de l'orthèse, selon un mode de réalisation,
la figure 4 est une vue arrière de l'orthèse de la figure 1, placée sur un membre inférieur droit,
les figures 5A, 5B représentent en coupe sagittale les os d'un membre inférieur droit (os iliaque, fémur, rotule, tibia), respectivement genou en extension et en flexion à 90°,
la figure 5C représente les figures 5A et 5B, superposées,
les figures 6A, 6B représentent une plaquette en gel polymère de l'orthèse dans deux configurations, respectivement suivant que le genou est en extension et en flexion,
la figure 7 représente une coupe transversale du genou droit,
les figures 8A, 8B sont des vues schématiques de l'orthèse en coupes transversale et longitudinale, suivant les plans AA' et BB' indiqués sur la figure 1, selon des modes de réalisation,
les figures 9A, 9B sont des vues schématiques de l'orthèse en coupes transversale et longitudinale, suivant les plans AA' et BB' indiqués sur la figure 1, selon d'autres modes de réalisation,
la figure 10 représente la plaquette en gel polymère selon un autre mode de réalisation,
les figures 11A, 11B et 11C sont des vues schématiques en coupes transversale et longitudinale suivant les plans AA' et BB' indiqués sur la figure 1, et de la face interne de l'orthèse, selon d'autres modes de réalisation.

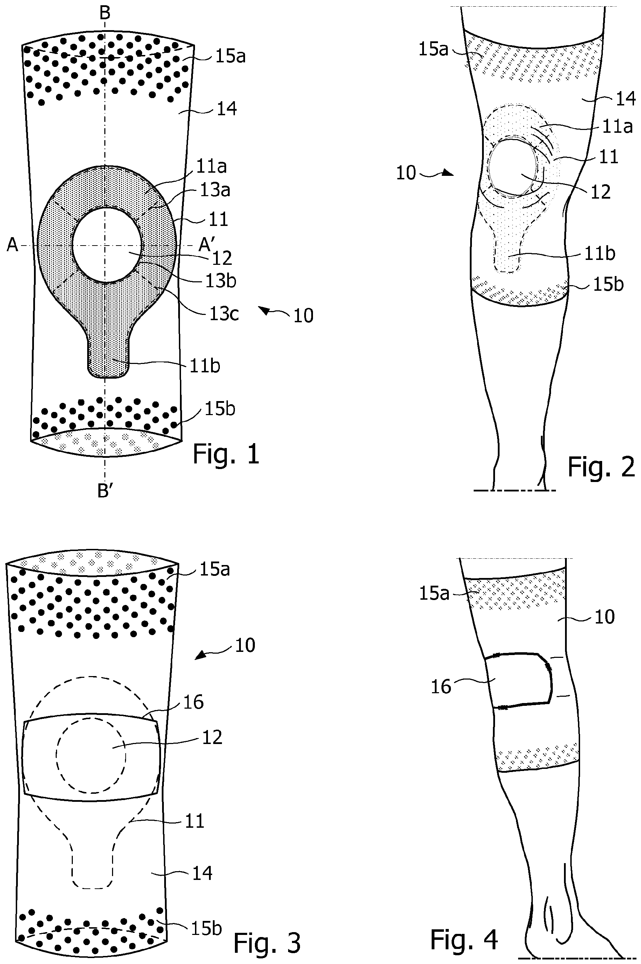

Les figures 1 et 2 représentent une orthèse de genou 10 selon un mode de réalisation, la figure 2 représentant l'orthèse mise en place sur un membre inférieur droit. La figure 1 représente l'orthèse dans une configuration retournée, la face visible étant celle destinée à venir en contact avec la peau. L'orthèse 10 comprend un manchon élastique 14 et une plaquette 11 comprenant une couche en un gel polymère viscoélastique, fixée sur la face du manchon 14 destinée à venir en contact avec la peau. Le manchon 14 est conformé pour exercer des forces de contention sur la cuisse, le genou et la jambe. A cet effet, le manchon 14 présente la forme d'un cylindre ayant un diamètre variable dans la direction longitudinale du manchon, adapté aux diamètres du bas de la cuisse, du genou et du haut de la jambe, de manière à obtenir des forces de contention souhaitées dans ces différentes parties du membre inférieur.

La plaquette 11 comprend une partie annulaire 11 a et une languette 11 b s'étendant depuis un bord extérieur de la partie annulaire. Sur la figure 2, la plaquette est fixée sur la face interne du manchon 14 de manière à ce que la partie annulaire et la languette viennent directement en contact avec la peau. La plaquette 11 est fixée en un emplacement du manchon 14 tel que la partie annulaire 11a puisse entourer la rotule, et la languette 11 b puisse couvrir la tubérosité tibiale antérieure (figure 2). La languette 11 b s'étend donc dans une direction distale du manchon 14. La partie annulaire 11a présente une ouverture 12 ayant des dimensions légèrement inférieures à celles de la rotule et une largeur entre les bords intérieur et extérieur (de la partie annulaire) comprise entre 2 et 4 cm. La languette 11 b présente des dimensions légèrement supérieures à celles de la tubérosité tibiale. Il est à noter que dans cette configuration, il peut ne pas être nécessaire de réaliser une orthèse différente pour les genoux droit et gauche, malgré le fait que la tubérosité tibiale antérieure ne soit pas centrée par rapport à un axe longitudinal de la cuisse passant par le centre de la rotule. La plaquette 11 sur la cuisse, le genou et la jambe peut présenter une épaisseur inférieure à 0,5 mm, par exemple comprise entre 0,35 et 0,45 mm. Le manchon 14 est conformé de manière à exercer des forces de contention conformes aux normes en vigueur.

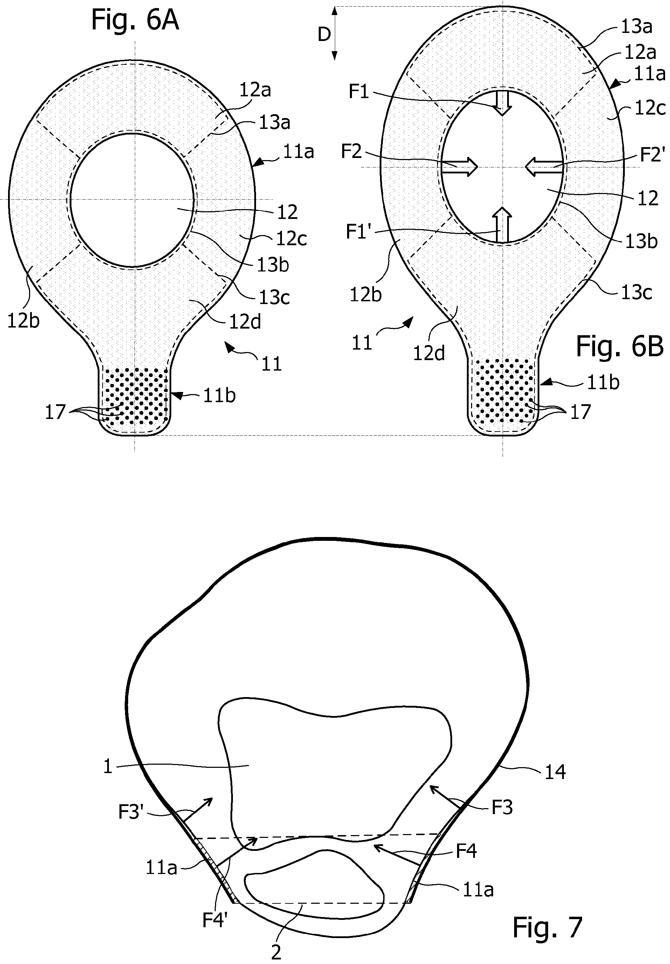

La plaquette 11 est fixée sur le manchon 14 par des lignes d'assemblage telles que des coutures. Dans l'exemple des figures 1, 2 et 6A, la plaquette 11 est fixée par des lignes d'assemblage 13a, 13b 13c. La ligne d'assemblage 13a est formée le long d'une partie proximale du bord externe de la partie annulaire 11a sur environ un quart de la circonférence de la partie annulaire. Les extrémités de la ligne d'assemblage 13a peuvent rejoindre le bord interne de la partie annulaire, mais cela n'est pas indispensable. La ligne d'assemblage 13b est formée le long de la totalité du bord interne de la partie annulaire 11a. La ligne d'assemblage 13c est formée le long d'une partie distale du bord externe de la plaquette 11 incluant le bord de la languette 11 b, sur environ un quart de la circonférence de la partie annulaire 11a. Les extrémités de la ligne d'assemblage 13c peuvent rejoindre le bord interne de la partie annulaire, mais cela n'est pas indispensable. En d'autres termes, les lignes d'assemblage 13a et 13c divisent la plaquette à partir de la partie annulaire en quatre secteurs 12a à 12d (figure 6A), à savoir un secteur proximal 12a fixé, un secteur distal fixé 12d, incluant la languette 11 b, et deux secteurs latéraux 12b, 12c non fixés le long du bord extérieur, s'étendant chacun sur environ un quart de la circonférence de la partie annulaire 11a. Les lignes d'assemblage 13a, 13c sont réalisées le long de tous les bords du secteur proximal 12a et du secteur distal 12d. Les bords externes des secteurs latéraux 12b, 12c sont donc laissés libres. A noter que la ligne d'assemblage 13b fixant le bord intérieur de la partie annulaire 11a au manchon sert simplement à éviter la formation de bâillements et peut donc être omise totalement, ou partiellement en conservant les parties situées sur les secteurs proximal 12a et distal 12d.

Au lieu d'être fixés par des lignes d'assemblage, le secteur proximal 12a et le secteur distal 12d de la plaquette 11 peuvent être fixés sur le manchon 14 par une couche de colle, les secteurs latéraux 12b, 12c n'étant pas collés sur le manchon 14.

Le manchon 14 peut être réalisé en un tissu tissé élastique suivant deux directions perpendiculaires, suivant la chaine et suivant la trame du tissu, par exemple un tissu en polyamide élasthanne. Ainsi, le tissu formant le manchon peut présenter un allongement maximal (suivant la chaine et la trame du tissu) compris entre 80 et 110%, par exemple égal à 95% et un module d'élasticité à 40% compris entre 5 et 7 N, par exemple égal à 6 N. Le tissu formant le manchon peut présenter une épaisseur inférieure à 0,5 mm, par exemple comprise entre 0,3 et 0,45 mm. Selon un mode de réalisation, le manchon 14 peut être réalisé de manière à couvrir la cuisse sur une longueur de 18 à 28 cm (à ±10% près) à partir de l'axe de la rotule.

Les lignes d'assemblage 13a, 13b, 13c peuvent être réalisées par des coutures. La ligne d'assemblage 13b peut être formée par un surjet.

La plaquette 11 présente une adhérence avec la peau telle que, sous l'effet des forces de contention exercées par le manchon 11, lorsque le manchon est étiré longitudinalement, elle reste étirée et applique localement à la peau des forces de traction parallèles à la surface de la peau, en direction du centre de la partie annulaire. L'ouverture 12 peut être refermée par une pièce en tissu tissé fin, qui peut être fixée à l'orthèse par la ligne d'assemblage 13b ou une autre ligne d'assemblage suivant le bord interne de la partie annulaire 11a.

Dans un mode de réalisation, la couche en gel polymère de la plaquette 11 est formée dans un gel de silicone obtenu par polymérisation au moins partielle d'un mélange d'huiles de silicone telles que des huiles de polydiméthylsiloxane. Un tel mélange permet d'obtenir une variété de gels de silicone présentant des propriétés différentes notamment de dureté, et d'adhésivité, en fonction des proportions respectives des huiles de silicone constituant le mélange, qui définissent le degré de polymérisation du mélange. Ainsi, en ajustant ces proportions, il est possible d'obtenir un gel viscoélastique plus ou moins dur et plus ou moins adhésif. L'ajustement de la dureté de la plaquette peut être effectué en tenant compte d'exigences d'élasticité et de résistance à l'usure sachant que la plaquette sera mécaniquement très sollicitée.

Selon un mode de réalisation, le manchon 14 comprend le long de ses bords proximal et distal, des bandes d'ancrage 15a, 15b assurant le maintien du manchon 14 sur la cuisse et sur la jambe, pour éviter que le manchon 14 glisse le long du membre inférieur, soit vers le bas, soit vers le haut. A cet effet, les bandes d'ancrage 15a, 15b comprennent des éléments présentant une certaine adhérence avec la peau. Les bandes d'ancrage 15a, 15b peuvent être réalisées en un tissu qui peut être le même que celui dans lequel est réalisé le manchon 14. Les éléments adhérents des bandes 15a, 15b peuvent se présenter sous la forme de plaquettes, de plots ou picots, par exemple réalisés en un gel polymère tel qu'un gel de silicone. La densité surfacique des éléments adhérents est prévue pour éviter un échauffement de la peau par frottement et cisaillement, pouvant conduire à des brûlures. Les éléments adhérents peuvent être plus larges le long des bords libres de la bande 15a et/ou 15b, que ceux formés le long des bords de la bande fixés au manchon 14.

L'orthèse peut être mise en place sur un membre inférieur en l'enfilant par le pied et en tirant sur le bord supérieur du manchon 14 jusqu'à ce que la plaquette 11 soit placée sur la rotule. La raideur naturelle du manchon fixe l'étirement du manchon, qui est très inférieur à la limite élastique du tissu formant le manchon. Il s'avère que l'allongement de la partie du manchon recouvrant la cuisse, lors d'une flexion du genou à 90°, reste inférieur à 20% dans la zone du manchon où la tension est maximum, et reste inférieur à 10% à 4 cm de cette zone vers la bande 15a. Cet allongement est très inférieur à l'allongement maximum du tissu formant le manchon. Dans ces conditions, le pliage alternatif du genou pendant la marche ou la course ne sollicite pas beaucoup le maintien du manchon sur la cuisse, réalisée par la bande 15a. Il en résulte que les bandes 15a, 15b sont suffisantes pour empêcher l'orthèse de glisser.

Il peut être constaté que les sollicitations élastiques du tissu du manchon 14 sont maximales juste au-dessus de la rotule et diminuent vers le haut de la cuisse. Le manchon 14 peut donc être prévu avec une longueur suffisante entre l'emplacement de la plaquette 11 et son bord proximal, de manière à placer la bande 15a dans une zone de la cuisse où les sollicitations élastiques du manchon 14 sont relativement faibles (zone de faible étirement musculaire). Selon un mode de réalisation, la bande 15a peut présenter une largeur plus importante que celle de la bande 15b, typiquement une largeur double de celle de la bande 15b. Ainsi, la bande 15a peut être réalisée à l'aide de plusieurs bandes plus étroites. Il en résulte l'avantage de permettre de s'adapter à la forte variation de diamètre de la cuisse dans cette zone, en donnant aux deux bandes formant la bande 15a des longueurs différentes. Dans le cas d'une réalisation de la bande 15a à l'aide de deux bandes cousues entre elles, l'une des deux bandes peut comprendre une rangée d'éléments adhérents en moins par rapport à l'autre bande formant la bande 15a, pour préserver une zone de couture des deux bandes entre elles, sans éléments adhérents.

Les figures 3 et 4 représentent la face arrière de l'orthèse, selon un mode de réalisation, la figure 4 représentant l'orthèse mise en place sur un membre inférieur droit. L'orthèse comprend une ouverture 16 formée sensiblement en regard de l'ouverture de la plaquette 11, en un emplacement correspondant au pli ou creux poplité du genou. L'ouverture 16 est fermée par une pièce en tissu tissé qui peut être fixée au manchon 14 par une couture. La pièce de tissu refermant l'ouverture 16 peut être élastique et présenter une épaisseur inférieure à 0,3 mm. Selon un mode de réalisation, la pièce de tissu tissé refermant l'ouverture 16 est réalisée dans le même tissu que celui refermant l'ouverture 12 de la plaquette 11. La pièce de tissu refermant l'ouverture 16 peut en outre être fixée au manchon tout autour de l'ouverture 16 de manière à rester en tension, quelle que soit la flexion du genou, sans introduire de jeu de forces parasite. De cette manière "l'envahissement" du poplité peut toujours être évitée.

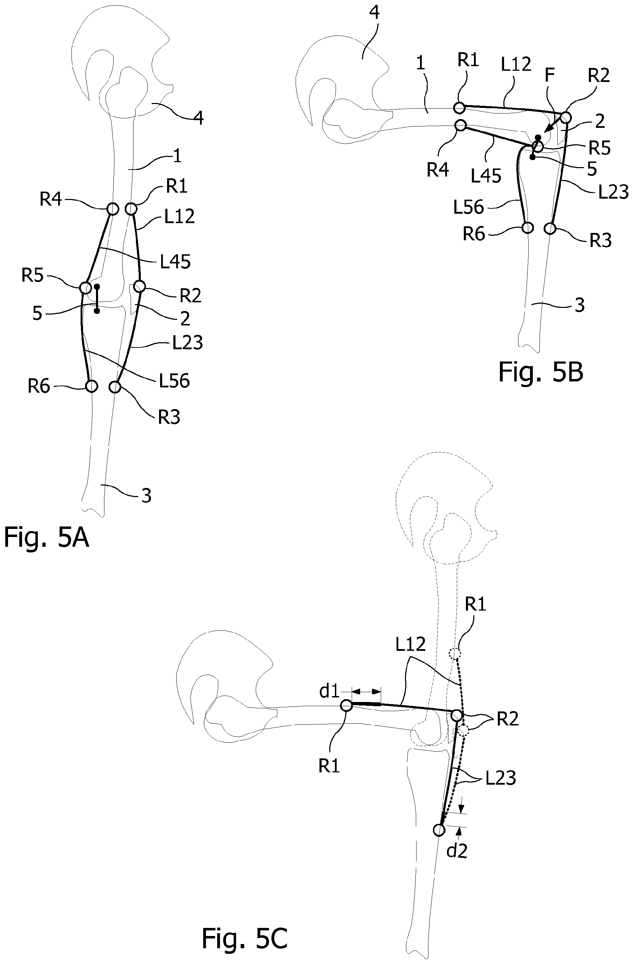

Les figures 5A, 5B représentent l'ossature d'un membre inférieur et du bassin en coupe sagittale (os iliaque 4, fémur 1, tibia 3 et rotule 2), dans des configurations genou en extension et genou fléchi à environ 90°. La figure 5C représente ces deux configurations, superposées. Les figures 5A, 5B symbolisent par des cercles des points repères antérieurs R1, R2, R3 et postérieurs R4, R5, R6 sur les os et par des lignes des tissus (muscles, tendons) L12, L23, L45, L56 liant ces points de repère. Les points R1 et R4 sont situés en une position proximale du fémur 1. Le point R2 est situé sur la rotule 2. Le point R5 est situé en une position distale du fémur 1. Les points R3 et R6 sont situés en une position distale du tibia 3. Ces figures montrent que les liaisons antérieures L12 entre les points R1 et R2 et L23 entre les points R2 et R3 subissent des étirements d1 et d2 entre les configurations tendue et fléchie du genou (figure 5C). En revanche, la liaison L45 entre les points R4 et R5 présente la même longueur dans ces deux configurations. La liaison R56 entre les points R5 et R6 présente également la même longueur dans ces deux configurations, mais est déformée dans la région du creux poplité dans la configuration genou fléchi à environ 90°. En effet, le fonctionnement de l'articulation du genou se traduit, entre le fémur 1 et le tibia 3, par un roulement et un glissement des condyles fémoraux sur les glènes tibiales et entre le fémur 1 et la rotule 2, et par un coulissement de la rotule 2 dans la trochlée du fémur 1. La prévision d'un tissu élastique fin 16 sur cette région particulière du creux poplité, permet d'éviter la formation de surépaisseurs résultant d'une superposition de plis de tissu, susceptibles d'induire une gêne ou une douleur lorsque le genou est fléchi en position assise ou accroupie. Au delà d'un millimètre d'épaisseur, ces surépaisseurs peuvent induire des irritations et même des brûlures. La prévision du tissu fin 16 constitue donc une amélioration importante pour ce type d'orthèse.

Les figures 5A, 5B représentent également les ligaments latéraux internes et externes 5 qui sont tendus lorsque le genou est en extension. Pendant une phase de flexion du genou, l'orthèse exerce une force F contribuant à maintenir la rotule 2 et à stabiliser latéralement le genou.

Sur la figure 4, l'ouverture 16 est sensiblement rectangulaire, les limites hautes et basses étant sensiblement rectilignes. Dans un mode de réalisation possible, la partie supérieure pourra être convexe de manière à suivre la forme cylindrique de la cuisse, et ainsi éviter l'apparition d'une gêne pour certaines anatomies.

L'orthèse telle que décrite précédemment peut présenter une masse de l'ordre de 50 g (à ±10%), à comparer avec celle des orthèses de l'art antérieur qui présentent couramment une masse supérieure à 150 g.

Les figures 6A, 6B représentent la plaquette 11 dans des configurations, respectivement non étirée et étirée, par exemple lorsque l'orthèse est en place sur un membre inférieur, respectivement, genou en extension et genou fléchi. En configuration non étirée, l'ouverture 12 de la plaquette 11 est sensiblement circulaire et adaptée à la forme de la rotule. La languette 11 b constitue un point d'ancrage sur la peau, obtenu par la conjonction de la force de contention exercée par le manchon 14 sur la languette 11, de la surface de la languette et en particulier de la languette 11b, et du coefficient d'adhérence (tack) du gel polymère formant la plaquette. La bande 15a constitue un point d'ancrage du manchon 14 sur la cuisse, et la bande 15b constitue un point d'ancrage du manchon sur la jambe. Durant l'étirement du manchon 14, lors d'une flexion du genou, une partie des étirements d1, d2 (figure 5C) est transmise à la plaquette en raison de son adhérence à la peau, de l'ancrage du manchon 14 par les bandes 15a, 15b, et d'un ancrage supplémentaire résultant de la liaison par la partie annulaire 11a de la plaquette à la rotule. Il en résulte un étirement D de la plaquette 11 et en particulier de la partie annulaire 11a. L'étirement D entraine une déformation élastique de la plaquette 11, provoquant notamment une déformation de l'ouverture 12 qui s'allonge suivant la direction longitudinale et se resserre suivant la direction transversale. Il en résulte l'apparition de forces de traction parallèles à la surface de la peau exercées par la plaquette sur la peau et sur le volume du membre qu'elle entoure. Ces forces comprennent des forces longitudinales F1, F1' opposées orientées vers le centre de l'ouverture 12 et des forces transversales F2, F2' opposées, également orientées vers le centre de l'ouverture 12. Les forces F2, F2' assurent le maintien de la rotule et évitent son débattement latéral.

Les forces F1, F1' participent au déroulement du pas lors de la marche ou la course. Ainsi, durant la marche ou la course, la partie annulaire 11a s'étire pendant une phase active où le pied est posé au sol tandis que l'inertie du corps participe à la flexion du genou. Pendant une phase passive où le pied ne repose plus sur le sol et le membre inférieur se tend vers l'avant pour prendre un nouvel appui, la partie annulaire 11 a reprend sa configuration non étirée et restitue donc à la jambe l'énergie élastique emmagasinée. Même si les forces F1, F1', F2, F2' sont relativement faibles, elles justifient le qualificatif de proprioceptif de l'orthèse. En effet, les forces F2, F2' sont suffisantes pour assurer un certain maintien de la rotule et soulager l'articulation en offrant la sensation que l'articulation est tenue. Quant aux forces F1 et F1', leur présence est perçue par l'intermédiaire du système musculo tendineux auquel elles s'appliquent.

La figure 7 représente une coupe transversale du genou droit et de l'orthèse l'enveloppant. Il peut être observé que la partie annulaire 11a prend une forme concave sensiblement tronconique, enveloppant les parties latérales droite, gauche, inférieure et supérieure de la rotule 2. La figure 7 montre également par des flèches les forces F3, F3', F4, F4' exercées par l'orthèse sur la rotule 2, ces forces étant exercées perpendiculairement à la surface du manchon 14 et de la plaquette 11. Les forces F3, F3' exercées par le manchon 14 sont dirigées vers le centre du genou, et les forces F4, F4' exercées par la partie annulaire 11a de la plaquette 11 sont dirigées sensiblement parallèlement à la surface de l'interface entre la rotule 2 et le fémur 1. L'orthèse permet ainsi d'assurer le maintien latéral de la rotule en particulier lorsque le genou est en extension ou au début d'un mouvement de flexion, c'est-à-dire dans des positions où les tissus qui l'enveloppent sont le moins tendus.

Il est à noter que l'extension longitudinale de la plaquette 11, en particulier de la partie annulaire 11a, est en partie facilitée par l'absence de coutures sur le bord externe des secteurs latéraux 12b, 12c de la partie annulaire 11a. A noter également que le manchon 14 peut présenter une capacité d'étirement longitudinal supérieure aux étirements d1+d2 (figure 5C), ceci afin d'éviter un glissement des bords proximal et/ou distal du manchon le long de la cuisse ou de la jambe.

Selon un mode de réalisation, la languette 11 b comporte des picots 17 pour augmenter son adhérence à la peau (figures 6A, 6B). Ces picots peuvent être formés par exemple lors de la fabrication par moulage de la plaquette 11.

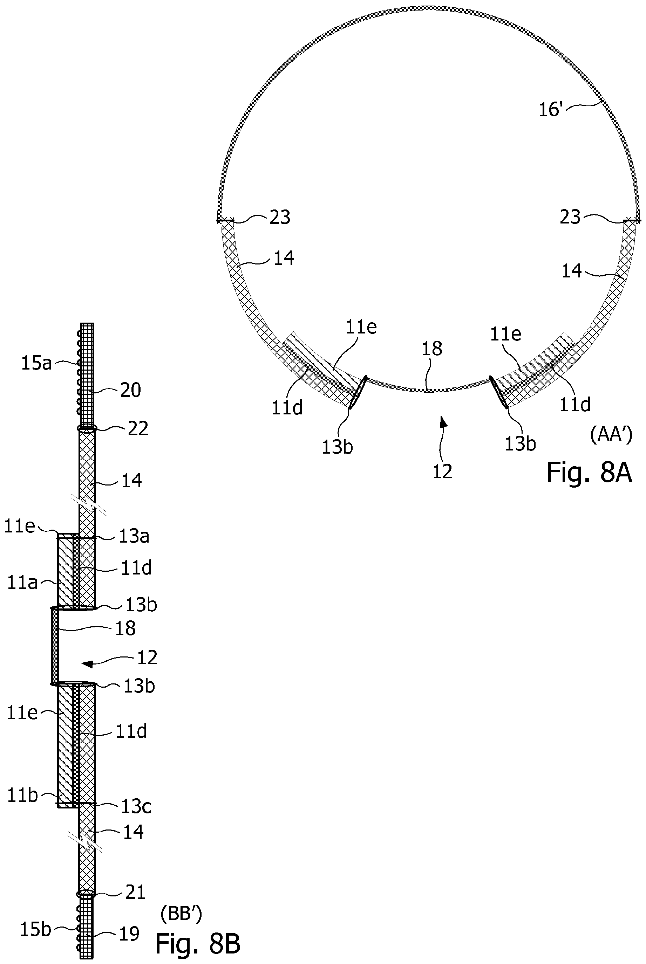

Les figures 8A, 8B représentent l'orthèse 10. Les figures 8A, 8B montrent en particulier la plaquette 11 comprenant la couche en gel polymère 11 e collée sur une pièce de tissu tissé élastique 11 d recouvrant l'ensemble d'une face de la couche en gel polymère, sans refermer l'ouverture 12 de la partie annulaire 11 a. L'ensemble de la couche en gel polymère 11e et de la pièce de tissu 11 d formant la plaquette 11, peut être ensuite cousu par les coutures 13a, 13b, 13c au manchon 14. Les coutures 13a et 13c peuvent être supprimées en collant la pièce de tissu 11 d (collée sur la couche en gel polymère 11e) au manchon 14, par une couche de colle répandue sur les secteurs annulaires proximal 12a et distal 12d (délimités par les coutures 13a et 13c). La pièce de tissu 11d peut être réalisée dans le même tissu que le manchon 14.

La dureté et l'épaisseur de la couche en gel polymère 11e de la plaquette 11 peuvent être choisies pour permettre de coudre l'ensemble de la plaquette 11 collée sur la pièce de tissu 11 d. Par ailleurs, l'adhérence ou le tack de la plaquette 11 peut être choisi afin d'éviter que cette dernière glisse sur la peau, compte tenu des forces de contention exercées par le manchon 14.

Des bandes 19, 20 assurant l'ancrage sur la peau des bords proximal et distal du manchon 14 peuvent être fixés par des coutures 21, 22 aux bords proximal et distal du manchon 14. Les bandes 19, 20 comportent par exemple des plaquettes ou des plots ou des picots 15a, 15b, ou encore des bandes, présentant une adhérence avec la peau supérieure à celle du manchon 14. Une ouverture 16 correspondant à la zone arrière du genou ménagée dans le manchon peut être refermée par une pièce de tissu tissé 16' qui est par exemple cousue par une couture 23, par exemple à plat sans que le manchon ou la pièce 16' soit préalablement tendus. La largeur de chacune des bandes 19, 20 et l'effet de contention exercé sur la cuisse ou sur la jambe peut être adaptée à la tenue souhaitée du manchon 14 sur le membre inférieur.

Selon un mode de réalisation, le manchon 14 ne comporte pas d'ouverture en regard de l'ouverture 12 de la plaquette 11.

Selon un autre mode de réalisation illustré par les figures 8A, 8B, l'ouverture 12 de la partie annulaire 11a est refermée par une pièce de tissu tissé 18 sensiblement aux dimensions de l'ouverture 12. La pièce 18 peut être cousue à plat sans que le manchon 14 ou la pièce 18 soient préalablement tendus. La pièce de tissu 18 peut être fixée par exemple du côté intérieur du manchon par la couture 13b ou bien une autre couture. La pièce de tissu 18 peut être élastique, et présenter une épaisseur inférieure à 0,25 mm, par exemple égale à 0,2 mm, un allongement maximal compris entre 50 et 90%, par exemple compris entre 60 et 80%, et un module d'élasticité à 40% compris entre 1 et 2 N. La pièce de tissu 18 peut être fixée sur le manchon 14 de manière à n'exercer aucune force sur sa périphérie lorsque le manchon n'est pas tendu. Lorsque le manchon 14 est tendu, il peut être souhaitable que la pièce de tissu 18 ne vienne pas contrecarrer l'extension de la plaquette 11, et qu'elle n'exerce sur la rotule que des forces non significatives. La pièce de tissu 18 présente un faible module élastique afin de seulement empêcher l'ouverture 12 de bailler dans certaines circonstances d'utilisation. Les coutures peuvent être réalisées de manière à ne pas entraver, ou entraver de manière limitée, l'étirement du manchon suivant son axe. Ainsi des formes de couture telles que le surjet plus lâche peuvent être préférées pour réaliser la fixation de la pièce de tissu 18.

Il est à noter que la présence de la pièce de tissu 18, si celle-ci déborde sur la plaquette 11, peut faciliter la formation de la couture 13b ou celle qui fixe la pièce 18. En effet, la présence de la pièce 18 peut permettre d'empêcher un contact direct la plaquette 11 et la machine à coudre utilisée pour réaliser cette couture, et donc d'éviter que l'adhérence de la plaquette entraine un blocage de l'orthèse dans la machine à coudre. Bien entendu, d'autres moyens tels que l'usage d'un lubrifiant adéquat, peuvent être utilisés pour éviter ces blocages. Il est également à noter que la couture 13b peut être réalisée sous la forme d'un simple piquage qui peut être retiré après fixation de la plaquette 11 sur le manchon 14.

Selon un autre mode de réalisation illustré par les figures 8A, 8B, le manchon 14 comporte l'ouverture 16 en regard de l'ouverture 12 de la plaquette 11. Une pièce de tissu tissé fin 16' peut refermer l'ouverture 16. La pièce de tissu 16' peut être réalisée dans le même tissu que la pièce 18.

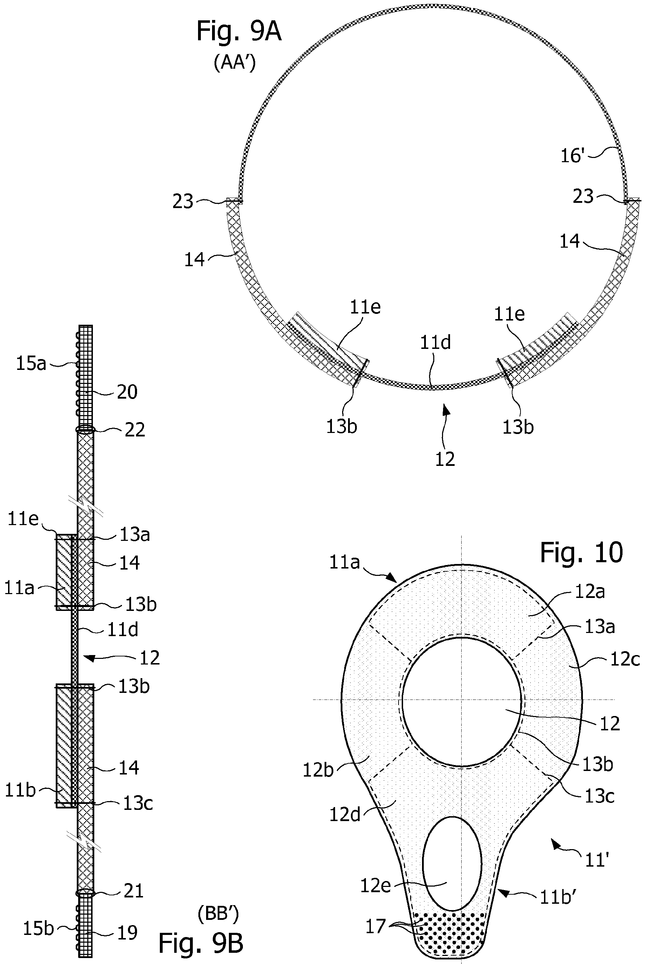

Les figures 9A, 9B représentent une orthèse selon un autre mode de réalisation. L'orthèse représentée sur les figures 9A, 9B diffère de celle représentée sur les figures 8A, 8B en ce que la pièce de tissu 11 d sur laquelle est fixée la couche en gel polymère 11e de la plaquette 11 n'est pas ouverte en regard de l'ouverture 12. La pièce de tissu 11 d referme donc l'ouverture 12. Il n'est donc pas nécessaire de prévoir la pièce 18. Comme précédemment, l'assemblage de la plaquette 11 et de la pièce de tissu 11d peut être effectué par collage. La plaquette 11 (couche en gel polymère 11e et pièce de tissu 11 d) peut être ensuite fixé au manchon 14 par les coutures 13a, 13b, 13c ou par une couche de colle répandue sur les secteurs proximal 12a et distal 12d délimités par les coutures 13a, 13b, 13c.

Par ailleurs, comme cela est montré sur la figure 9A, la pièce de tissu 16' peut être fixée côté intérieur du manchon 14.

La figure 10 représente une plaquette 11' selon un autre mode de réalisation. La plaquette 11' diffère de celle représentée en particulier sur les figures 6A, 6B en ce qu'elle comprend une languette 11 b' comportant une ouverture 12e à l'emplacement de la tubérosité tibiale antérieure. Comme la tubérosité tibiale antérieure n'est pas dans située sur un axe longitudinal de la cuisse passant par le centre la rotule, l'ouverture 12e peut être légèrement désaxée. Il en résulte que l'orthèse sera différente pour les genoux droit et gauche. Comme précédemment, la languette 11 b' peut comporter des picots 17 prévus pour augmenter l'adhérence de la languette sur la peau en présence des forces de contention exercées par le manchon 14. Il peut être également prévu d'élargir l'ouverture 12c afin d'éviter d'avoir à appairer l'orthèse.

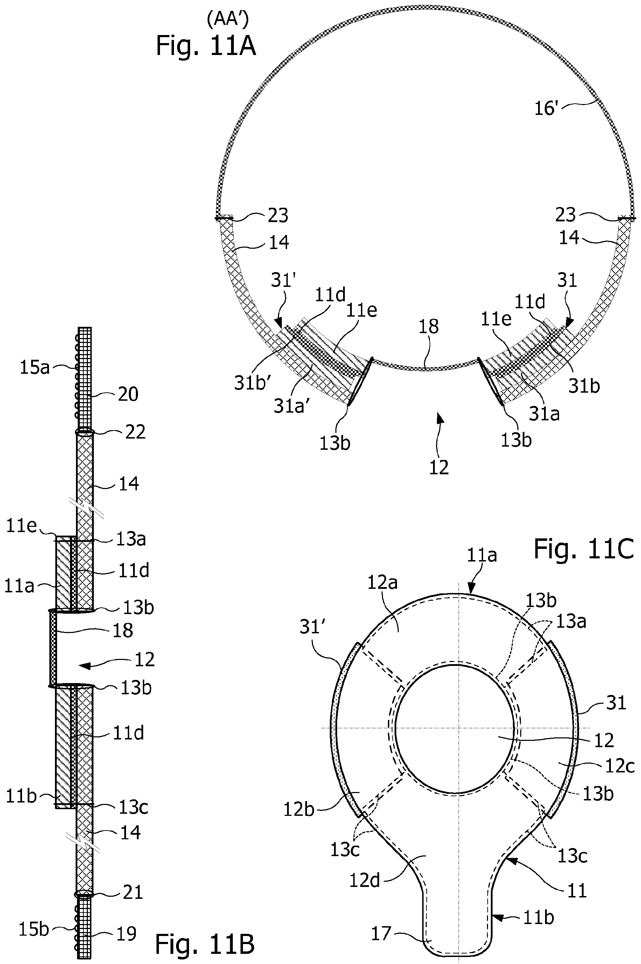

Les figures 11A, 11B et 11C représentent une orthèse selon d'autres modes de réalisation. L'orthèse représentée sur les figures 11A, 11B et 11C diffère de celle représentée sur les figures 8A, 8B en ce qu'elle comprend en plus de la plaquette 11, des coussinets latéraux 31, 31' comprenant une couche en gel polymère viscoélastique 31 a, 31a' collée sur une couche en tissu tissé élastique 31 b, 31 b'. Les coussinets 31, 31' présentent sensiblement la forme des secteurs annulaires latéraux 12b, 12c de la plaquette 11. Les coussinets 31, 31' sont fixés sous les secteurs latéraux 12b, 12c de la plaquette 11, de chaque côté de l'ouverture 12 de la partie annulaire 11 a. La figure 11 B est identique à la figure 8B, les coussinets 31, 31' n'étant pas situés sur l'axe longitudinal de la plaquette 11. Les coussinets 31, 31' sont fixés au manchon 14 à leurs extrémités par des lignes d'assemblage s'étendant radialement par rapport à la plaquette 11. Ainsi, les coussinets 31, 31' peuvent être fixés au manchon 14 en même temps que la plaquette 11 uniquement par les parties radiales des lignes d'assemblage 13a et 13c (et non pas par la ligne d'assemblage centrale 13b le long de l'ouverture 12). Des bords latéraux des coussinets 31, 31' sont donc laissés libres. Les coussinets 31, 31' peuvent dépasser latéralement légèrement du bord externe de la plaquette 11.

Sur la figure 11A, chacun des coussinets 31, 31' comprend une couche en gel polymère 31 a, 31 a' préalablement collée sur une couche de tissu 31 b, 31 b'. Les coussinets 31, 31' sont disposés sous la plaquette 11 de manière à ce que les couches en tissu 31 b, 31 b' soient disposées contre la couche de tissu 11 d de la plaquette 11. Ainsi, les couches en gel polymère de la plaquette 11 et des coussinets 31 ou 31' sont séparées par les deux couches de tissu 11d, et 31 b ou 31 b'. Les coussinets peuvent être formés dans le même matériau que la plaquette 11 et peuvent présenter une même épaisseur que cette dernière.

A noter que les couches de tissu 31 b, 31b'peuvent être supprimées, de sorte que les deux couches 31 a, 31a' sont collées sur la face de la couche de tissu 11 d non recouverte par la couche en gel polymère 11 e.

De part et d'autre de la plaquette 11, sous les bords latéraux non cousus de cette dernière, il peut être prévu de former des pinces (ou embus) dans le manchon 14 pour permettre au tissu du manchon de se déployer sur la courbure du genou, malgré la surépaisseur formée par la plaquette à double épaisseur, sans appliquer des forces de compression excessives liées à la courbure à absorber du genou.

La prévision des coussinets 31, 31' permet de maintenir plus fermement la rotule latéralement. En effet, il peut être souhaitable de soulager davantage les ligaments, notamment lorsque ceux-ci sont fortement sollicités par exemple durant la pratique de certains sports comme le ski alpin. Lors de la pratique de ce sport, les genoux subissent des efforts latéraux en position plus ou moins fléchie, en virage ou pour absorber des irrégularités du sol.

Il apparaîtra clairement à l'homme de l'art que la présente invention est susceptible de diverses variantes de réalisation et diverses applications. En particulier, l'invention ne s'applique pas seulement à l'articulation du genou, mais également à d'autres articulations telles que le coude, l'épaule, ou encore le pouce. La forme et les dimensions de l'orthèse, en particulier du manchon et de la plaquette, sont alors adaptées à la configuration de l'articulation dont un maintien est à assurer. Les adaptations ainsi requises pour tenir compte de spécificités de l'articulation qui ne comporte pas nécessairement de rotule, sont mineures et à la portée de l'homme du métier. Toutefois, quelle que soit l'articulation à laquelle elle est destinée, la plaquette comprend une partie annulaire et une languette s'étendant depuis un bord de la partie annulaire. L'ouverture de la partie annulaire est adaptée aux dimensions d'une zone proéminente de l'articulation à maintenir. La languette est prévue pour s'appliquer sur une zone de la peau recouvrant des tissus plus durs que ceux couverts par la partie annulaire de la plaquette. La forme de la languette peut être adaptée à la forme des tissus au dessus desquels la languette est disposée, pour obtenir le meilleur ancrage possible. Ainsi, la languette de la plaquette peut présenter une longueur de 4 à 20 cm, et une largeur de 2 cm à la largeur de la partie annulaire. Pour certaines articulations comme par exemple l'épaule, il peut être prévu deux languettes s'étendant respectivement de part et d'autre de la partie annulaire.

Par ailleurs, les bandes de maintien 15a, 15b peuvent être supprimées partiellement ou en totalité, en remplaçant le manchon par un vêtement de type corsaire ou fuseau serré (pour le genou), couvrant le bassin et au moins un membre inférieur de l'utilisateur devant être maintenu par l'orthèse. Le maintien du vêtement à la cheville peut être assuré en exploitant la forme en fuseau de la jambe au voisinage de la cheville.

## Revendications

1. Orthèse comprenant un manchon (14) en un tissu tissé élastique, conformé pour exercer des forces de contention sur un membre de part et d'autre et sur une articulation, et une plaquette (11, 11') comprenant une couche en un gel polymère viscoélastique (11e), la couche en gel polymère étant fixée sur une face interne du manchon pour venir en contact direct avec la peau sur l'articulation,
**caractérisé en ce que** la plaquette (11, 11') comprend une couche en un tissu élastique (11d) sur laquelle est collée la couche en gel polymère (11e), la couche en gel polymère comprenant une partie annulaire (11a) conformée pour entourer le sommet d'une zone proéminente de l'articulation, et une languette (11b, 11 b') s'étendant depuis un bord extérieur de la partie annulaire, dans une direction axiale du manchon, la couche en gel polymère présentant une adhérence avec la peau telle que, sous l'effet des forces de contention exercées par le manchon, lorsque le manchon est étiré longitudinalement, elle reste étirée et applique localement à la partie de membre sous-jacente des forces de maintien en direction du centre de la zone proéminente de l'articulation, et des forces de rappel dans l'axe du membre.

2. Orthèse selon la revendication 1, dans laquelle la plaquette (11, 11') est fixée au manchon (14) seulement par un secteur proximal (12a) de la partie annulaire (11a) et par une partie incluant un secteur distal (12d) de la partie annulaire et la languette (11 b, 11 b').

3. Orthèse selon la revendication 1 ou 2, dans laquelle la plaquette (11, 11') est fixée au manchon (14) de manière à laisser libre des bords latéraux externes de la partie annulaire (11a) de la plaquette.

4. Orthèse selon l'une des revendications 1 à 3, dans laquelle la plaquette (11, 11') est fixée au manchon (14) le long d'un bord intérieur de la partie annulaire (11a).

5. Orthèse selon l'une des revendications 1 à 4, comprenant deux coussinets latéraux (31, 31') comprenant une couche gel polymère viscoélastique (31 a, 31 a') et présentant la forme de secteurs latéraux de la plaquette (11) fixées par des lignes d'assemblage s'étendant radialement par rapport à la plaquette, sous des secteurs latéraux (12b, 12c) de la plaquette, des bords latéraux des coussinets étant laissés libres.

6. Orthèse selon l'une des revendications 1 à 5, dans laquelle la partie annulaire (11a) de la plaquette (11, 11') présente une ouverture (12) coïncidant avec une première ouverture formée dans le manchon (14), l'ouverture étant refermée par une pièce (18, 11 d) en un tissu tissé élastique d'épaisseur inférieure à 0,3 mm fixée sur le manchon et/ou sur la plaquette.

7. Orthèse selon l'une des revendications 1 à 6, dans laquelle le manchon (14) comprend une seconde ouverture (16) dans une zone diamétralement opposée à la plaquette (11, 11') et refermée par une pièce (16') en un tissu tissé élastique d'épaisseur inférieure à 0,3 mm.

8. Orthèse selon la revendication 7, dans laquelle la pièce (16') refermant la seconde ouverture (16) formée dans le manchon (14), est fixée au manchon de manière à rester tendue quelle que soit la flexion de l'articulation.

9. Orthèse selon l'une des revendications 1 à 8, dans laquelle le manchon (14) comporte le long de bords proximal et distal, sur sa face destinée à venir en contact avec la peau, des éléments adhérents (15a, 15b) pour contribuer au maintien du manchon sur le membre.

10. Orthèse selon la revendication 9, dans laquelle les éléments adhérents (15a, 15b) sont formés sur des bandes (19, 20), fixées aux bords proximal et distal du manchon (14), les bandes étant réalisées en un tissu élastique pouvant être le même que celui du manchon (14).

11. Orthèse selon la revendication 9 ou 10, dans laquelle la bande d'éléments adhérents fixée au bord proximal du manchon (14) comprend deux bandes fixées l'une à l'autre et de longueurs différentes pour s'adapter à la forme du membre.

12. Orthèse selon l'une des revendications 1 à 11, dans laquelle le manchon (14) présente une épaisseur inférieure à 0,5 mm, et la plaquette (11, 11') une épaisseur inférieure à 1 mm.

13. Procédé de fabrication d'une orthèse selon l'une des revendications précédentes comprenant des étapes consistant à :
former un manchon (14) en un tissu tissé élastique, apte à exercer des forces de contention sur un membre de part et d'autre et sur une articulation,
former une plaquette (11, 11') comprenant une couche (11e) en un gel polymère viscoélastique, une couche en un tissu élastique (11d) collée sur la couche en gel polymère, la couche en gel polymère comprenant une partie annulaire (11a) conformée pour entourer le sommet de l'articulation en position fléchie, et une languette (11b, 11 b') s'étendant depuis un bord extérieur de la partie annulaire, et
fixer la plaquette sur une face interne du manchon pour que la couche en gel polymère vienne en contact direct avec la peau sur l'articulation, la languette étant orientée dans une direction distale du manchon, la couche en gel polymère présentant une adhérence avec la peau telle que, sous l'effet des forces de contention exercées par le manchon, lorsque le manchon est étiré longitudinalement, elle reste étirée et applique localement à la peau des forces de traction parallèles à la surface de la peau, en direction du centre de la partie annulaire.

14. Procédé de fabrication selon la revendication 13, dans lequel la plaquette (11, 11') est fixée au manchon (14) par une couture (13a, 13b, 13c) de manière à ce que des parties latérales des bords externes de la partie annulaire (11a) soient laissées libres.

15. Procédé de fabrication selon la revendication 14, comprenant une étape de fixation par des lignes d'assemblage (13a, 13c) s'étendant radialement par rapport à la plaquette (11), de coussinets (31, 31') comprenant une couche en gel polymère viscoélastique (31 a, 31a') présentant la forme de secteurs latéraux de la plaquette, sous des parties latérales (12b, 12c) de la partie annulaire (11 a) de la plaquette (11), des bords latéraux des coussinets étant laissés libres.

16. Procédé de fabrication selon l'une des revendications 13 à 15, comprenant une étape de fixation d'une pièce de tissu tissé élastique (18, 11 d) pour refermer l'ouverture de la partie annulaire (11a) de la plaquette (11, 11'), la pièce de tissu présentant une épaisseur inférieure à 0,3 mm.

17. Procédé de fabrication selon l'une des revendications 13 à 16, comprenant des étapes de formation d'une ouverture (16) dans le manchon, en regard de la plaquette (11, 11'), et de fixation d'une pièce de tissu tissé élastique (16') pour refermer l'ouverture formée dans le manchon, la pièce de tissu présentant une épaisseur inférieure à 0,3 mm.

18. Procédé de fabrication selon la revendication 17, dans lequel la pièce (16') refermant la seconde ouverture (16) formée dans le manchon (14), est fixée au manchon de manière à rester tendue quelle que soit la flexion du genou.

19. Procédé de fabrication selon l'une des revendications 13 à 18, comprenant une étape de moulage de la plaquette (11, 11'), le moule formant des picots (17) sur la face de la languette (11 b, 11 b') destinée à venir en contact avec la peau, pour augmenter l'adhérence de la languette sur la peau.

20. Procédé de fabrication selon l'une des revendications 13 à 19, comprenant des étapes de fixation le long des bords proximal et distal du manchon (14) de bandes (19, 20) en tissu élastique, comportant des éléments (15a, 15b) adhérents pour contribuer au maintien du manchon sur le membre.

## Patentansprüche

1. Orthese umfassend eine Hülse (14) aus einem elastischen Gewebe, die geformt ist, um Stützkräfte auf beide Seiten eines Körpergliedes und auf ein Gelenk auszuüben, und ein Plättchen (11, 11') umfassend eine Schicht aus einem viskoelastischen Polymergel (11e), wobei die Schicht aus Polymergel an einer Innenseite der Hülse befestigt ist, um unmittelbar mit der Haut am Gelenk in Kontakt zu kommen,
**dadurch gekennzeichnet, dass** das Plättchen (11, 11') eine Schicht aus einem elastischen Gewebe (11 d) umfasst, an der die Schicht aus Polymergel (11 e) angeklebt ist, wobei die Schicht aus Polymergel einen ringförmigen Teil (11a) umfasst, der so geformt ist, dass er den obersten Teil einer vorspringenden Zone des Gelenks umgibt, und eine Lasche (11 b, 11 b'), die sich von einem Außenrand des ringförmigen Teils in eine axiale Richtung der Hülse erstreckt, wobei die Schicht aus Polymergel eine solche Haftung an der Haut aufweist, dass unter der Wirkung der beim Längsstrecken der Hülse von der Hülse ausgeübten Stützkräfte sie im gestreckten Zustand bleibt und Haltekräfte lokal auf den darunterliegenden Körpergliedteil in Richtung auf das Zentrum der vorspringenden Zone des Gelenks und Rückstellkräfte in der Achse des Körpergliedes ausübt.

2. Orthese nach Anspruch 1, bei der das Plättchen (11, 11') an der Hülse (14) nur mit einem proximalen Bereich (12a) des ringförmigen Teils (11a) und mit einem Teil, der einen distalen Bereich (12d) des ringförmigen Teils und die Lasche (11 b, 11 b') umfasst, befestigt ist.

3. Orthese nach Anspruch 1 oder 2, bei der das Plättchen (11, 11') an der Hülse (14) so befestigt ist, dass äußere Seitenkanten des ringförmigen Teils (11 a) des Plättchens frei gelassen werden.

4. Orthese nach einem der Ansprüche 1 bis 3, bei der das Plättchen (11, 11') an der Hülse (14) entlang einem inneren Rand des ringförmigen Teils (11a) befestigt ist.

5. Orthese nach einem der Ansprüche 1 bis 4, umfassend zwei Seitenpolster (31, 31'), die eine viskoelastische Schicht aus Polymergel (31 a, 31 a') umfassen und die Form von Seitenbereichen des Plättchens (11) aufweisen, und die durch sich radial zum Plättchen erstreckende Verbindungslinien unter Seitenbereichen (12b, 12c) des Plättchens befestigt sind, wobei Seitenkanten der Polster frei gelassen werden.

6. Orthese nach einem der Ansprüche 1 bis 5, bei der der ringförmige Teil (11a) des Plättchens (11, 11') eine Öffnung (12) aufweist, die mit einer ersten in der Hülse (14) ausgebildeten Öffnung übereinstimmt, wobei die Öffnung von einem an der Hülse und/oder am Plättchen befestigten Stück (18, 11d) aus einem elastischen Gewebe mit einer Dicke von weniger als 0,3 mm verschlossen wird.

7. Orthese nach einem der Ansprüche 1 bis 6, bei der die Hülse (14) eine zweite Öffnung (16) in einer dem Plättchen (11, 11') diametral gegenüberliegenden Zone umfasst, die von einem Stück (16') aus einem elastischen Gewebe mit einer Dicke von weniger als 0,3 mm verschlossen wird.

8. Orthese nach Anspruch 7, bei der das die zweite in der Hülse (14) ausgebildete Öffnung (16) verschließende Stück (16') an der Hülse so befestigt ist, dass es unabhängig von der Beugung des Gelenks gespannt bleibt.

9. Orthese nach einem der Ansprüche 1 bis 8, bei der die Hülse (14) auf ihrer mit der Haut in Kontakt zu kommenden Seite haftende Elemente (15a, 15b) entlang proximalen und distalen Kanten aufweist, um zum Halt der Hülse am Körperglied beizutragen.

10. Orthese nach Anspruch 9, bei der die haftenden Elemente (15a, 15b) auf Streifen (19, 20) gebildet werden, die an den proximalen und distalen Kanten der Hülse (14) befestigt sind, wobei die Streifen aus einem elastischen Gewebe ausgeführt werden, das mit demjenigen der Hülse (14) identisch sein kann.

11. Orthese nach Anspruch 9 oder 10, bei der der Streifen haftender Elemente, der an der proximalen Kante der Hülse (14) befestigt ist, zwei Streifen umfasst, die aneinander befestigt und von verschiedenen Längen sind, um sich an die Form des Körpergliedes anzupassen.

12. Orthese nach einem der Ansprüche 1 bis 11, bei der die Hülse (14) eine Dicke von weniger als 0,5 mm aufweist und das Plättchen (11, 11') eine Dicke von weniger als 1 mm aufweist.

13. Verfahren zum Herstellen einer Orthese nach einem der vorhergehenden Ansprüche, umfassend Schritte, die darin bestehen:
eine Hülse (14) aus einem elastischen Gewebe zu bilden, die Stützkräfte auf beide Seiten eines Körpergliedes und auf ein Gelenk ausüben kann,
ein Plättchen (11, 11') zu bilden, umfassend eine Schicht (11e) aus einem viskoelastischen Polymergel, eine an der Schicht aus Polymergel angeklebte Schicht aus einem elastischen Gewebe (11d), wobei die Schicht aus Polymergel einen ringförmigen Teil (11a) umfasst, der so geformt ist, dass er den obersten Teil des Gelenks in gebeugter Position umgibt, und eine Lasche (11 b, 11 b'), die sich von einem Außenrand des ringförmigen Teils erstreckt, und
das Plättchen an einer Innenseite der Hülse so zu befestigen, dass die Schicht aus Polymergel unmittelbar mit der Haut am Gelenk in Kontakt kommt, wobei die Lasche in einer distalen Richtung der Hülse ausgerichtet ist, wobei die Schicht aus Polymergel eine solche Haftung an der Haut aufweist, dass unter der Wirkung der beim Längsstrecken der Hülse von der Hülse ausgeübten Stützkräfte sie im gestreckten Zustand bleibt und Zugkräfte lokal auf die Haut parallel zur Hautoberfläche in Richtung auf das Zentrum des ringförmigen Teils ausübt.

14. Herstellungsverfahren nach Anspruch 13, bei dem das Plättchen (11, 11') an der Hülse (14) durch eine Naht (13a, 13b, 13c) so befestigt ist, dass seitliche Teile der Außenkanten des ringförmigen Teils (11a) frei gelassen werden.

15. Herstellungsverfahren nach Anspruch 14, umfassend einen Schritt, der darin besteht, unter seitlichen Teilen (12b, 12c) des ringförmigen Teils (11a) des Plättchens (11), durch sich radial zum Plättchen (11) erstreckende Verbindungslinien (13a, 13c) Polstern (31, 31') zu befestigen, die eine Schicht aus einem viskoelastischen Polymergel (31a, 31a') umfassen und die die Form von Seitenbereichen des Plättchens aufweisen, wobei Seitenkanten der Polster frei gelassen werden.

16. Herstellungsverfahren nach einem der Ansprüche 13 bis 15, umfassend einen Schritt des Befestigens eines Stücks aus einem elastischen Gewebe (18, 11d), um die Öffnung des ringförmigen Teils (11a) des Plättchens (11, 11') zu verschließen, wobei das Gewebestück eine Dicke von weniger als 0,3 mm aufweist.

17. Herstellungsverfahren nach einem der Ansprüche 13 bis 16, umfassend Schritte des Ausbildens einer Öffnung (16) in der Hülse gegenüber dem Plättchen (11, 11'), und des Befestigens eines Stücks aus einem elastischen Gewebe (16'), um die in der Hülse ausgebildete Öffnung zu verschließen, wobei das Gewebestück eine Dicke von weniger als 0,3 mm aufweist.

18. Herstellungsverfahren nach Anspruch 17, bei dem das Stück (16'), das die zweite in der Hülse (14) ausgebildete Öffnung (16) verschließt, an der Hülse so befestigt ist, dass es unabhängig von der Kniebeugung gespannt bleibt.

19. Herstellungsverfahren nach einem der Ansprüche 13 bis 18, umfassend einen Schritt des Formens des Plättchens (11, 11'), wobei die Form Noppen (17) auf der mit der Haut in Kontakt zu kommenden Laschenseite (11 b, 11 b') bildet, um die Haftung der Lasche an der Haut zu erhöhen.

20. Herstellungsverfahren nach einem der Ansprüche 13 bis 19, umfassend Schritte, die darin bestehen, entlang den proximalen und distalen Kanten der Hülse (14) Streifen (19, 20) aus elastischem Gewebe zu befestigen, die haftende Elemente (15a, 15b) umfassen, um zum Halt der Hülse am Körperglied beizutragen.

## Claims

1. Orthosis comprising a sleeve (14) made of a woven elastic fabric, shaped to exert compression forces on a member on either side of and on a joint, and a plate (11, 11') comprising a layer of a viscoelastic polymer gel (11e),
the layer of polymer gel being fixed onto an inner face of the sleeve to come into direct contact with the skin on the joint,
**characterized in that** the plate (11, 11') comprises a layer made of an elastic fabric (11d) onto which the layer of polymer gel (11e) is adhered, the layer of polymer gel
comprising an annular part (11a) shaped to surround the top of a protruding area of the joint, and a tab (11 b, 11 b') extending from an outer edge of the annular part, in an axial direction of the sleeve, the layer of polymer gel adhering to the skin such that, under the effect of the compression forces exerted by the sleeve, when the sleeve is stretched longitudinally, the layer of polymer gel remains stretched and applies support forces locally to the underlying member part, toward the center of the protruding area of the joint, and return forces along the axis of the member.

2. Orthosis according to claim 1, wherein the plate (11, 11') is only fixed to the sleeve (14) by a proximal sector (12a) of the annular part (11a) and by a part including a distal sector (12d) of the annular part and the tab (11 b, 11 b').

3. Orthosis according to claim 1 or 2, wherein the plate (11, 11') is fixed to the sleeve (14) so as to leave outer side edges of the annular part (11a) of the plate free.

4. Orthosis according to one of claims 1 to 3, wherein the plate (11, 11') is fixed to the sleeve (14) along an inner edge of the annular part (11 a).

5. Orthosis according to one of claims 1 to 4, comprising two lateral pads (31, 31') comprising a layer of viscoelastic polymer gel (31 a, 31 a') and having the shape of lateral sectors of the plate (11) fixed by assembly lines extending radially in relation to the plate, beneath lateral sectors (12b, 12c) of the plate, side edges of the pads being left free.

6. Orthosis according to one of claims 1 to 5, wherein the annular part (11 a) of the plate (11, 11') has an opening (12) coinciding with a first opening formed in the sleeve (14), the opening being closed by a piece (18, 11d) made of a woven elastic fabric less than 0.3 mm thick fixed onto the sleeve and/or onto the plate.

7. Orthosis according to one of claims 1 to 6, wherein the sleeve (14) comprises a second opening (16) in an area diagonally opposite the plate (11, 11') and closed by a piece (16') made of a woven elastic fabric less than 0.3 mm thick.

8. Orthosis according to claim 7, wherein the piece (16') closing the second opening (16) formed in the sleeve (14) is fixed to the sleeve so as to remain taut whatever the bending of the joint.

9. Orthosis according to one of claims 1 to 8, wherein the sleeve (14) comprises along proximal and distal edges, on its face intended to come into contact with the skin, adherent elements (15a, 15b) to contribute to the hold of the sleeve on the member.

10. Orthosis according to claim 9, wherein the adherent elements (15a, 15b) are formed on straps (19, 20), fixed to the proximal and distal edges of the sleeve (14), the straps being made of an elastic fabric that may be the same as the fabric of the sleeve (14).

11. Orthosis according to claim 9 or 10, wherein the strap of adherent elements fixed to the proximal edge of the sleeve (14) comprises two straps fixed together and of different lengths to adapt to the shape of the member.

12. Orthosis according to one of claims 1 to 11, wherein the sleeve (14) is less than 0.5mm thick, and the plate (11, 11') is less than 1 mm thick.

13. Method for manufacturing an orthosis according to one of the previous claims comprising steps of:
forming a sleeve (14) from a woven elastic fabric, capable of exerting compression forces on a member on either side of and on a joint,
forming a plate (11, 11') comprising a layer (11e) of a viscoelastic polymer gel, a layer made of an elastic fabric (11 d) adhered onto the layer of polymer gel, the layer of polymer gel comprising an annular part (11a) shaped to surround the top of the joint in flexed position, and a tab (11 b, 11 b') extending from an outer edge of the annular part, and
fixing the plate onto an inner face of the sleeve so that the layer of polymer gel comes into direct contact with the skin on the joint, the tab being oriented in a distal direction of the sleeve, the layer of polymer gel adhering to the skin such that, under the effect of the compression forces exerted by the sleeve, when the sleeve is stretched longitudinally, the layer of polymer gel remains stretched and applies tensile forces locally to the skin parallel to the surface of the skin, toward the center of the annular part.

14. Manufacturing method according to claim 13, wherein the plate (11, 11') is fixed to the sleeve (14) by a seam (13a, 13b, 13c) so that lateral parts of the external edges of the annular part (11 a) are left free.

15. Manufacturing method according to claim 14, comprising a step of fixing by assembly lines (13a, 13c) extending radially in relation to the plate (11), pads (31, 31') comprising a layer of viscoelastic polymer gel (31 a, 31 a') having the shape of lateral sectors of the plate, beneath lateral parts (12b, 12c) of the annular part (11 a) of the plate (11), side edges of the pads being left free.

16. Manufacturing method according to one of claims 13 to 15, comprising a step of fixing a piece of woven elastic fabric (18, 11 d) to close the opening of the annular part (11 a) of the plate (11, 11'), the piece of fabric being less than 0.3 mm thick.

17. Manufacturing method according to one of claims 13 to 16, comprising steps of forming an opening (16) in the sleeve, opposite the plate (11, 11'), and of fixing a piece of woven elastic fabric (16') to close the opening formed in the sleeve, the piece of fabric being less than 0.3 mm thick.

18. Manufacturing method according to claim 17, wherein the piece (16') closing the second opening (16) formed in the sleeve (14), is fixed to the sleeve so as to remain taut whatever the bending of the knee.

19. Manufacturing method according to one of claims 13 to 18, comprising a step of molding the plate (11, 11'), the mold forming spikes (17) on the face of the tab (11 b, 11 b') intended to come into contact with the skin, to increase the adhesion of the tab to the skin.

20. Manufacturing method according to one of claims 13 to 19, comprising steps of fixing, along the proximal and distal edges of the sleeve (14), straps (19, 20) made of elastic fabric, comprising adherent elements (15a, 15b) to contribute to the hold of the sleeve on the member.
